# EUROPEAN PATENT SPECIFICATION

(11) **EP 3 628 000 B1**
(45) Date of publication and mention of the grant of the patent: **01.12.2021**
(21) Application number: 18725892.6
(22) Date of filing: 10.04.2018
(51) Int. Cl.: A61F 13/02

(54) **WOUND DRESSING**
WUNDAUFLAGE
PANSEMENT POUR PLAIES

(30) Priority: 11.04.2017 GB 201705800; 04.07.2017 GB 201710771
(43) Date of publication of application: 01.04.2020
(73) Proprietor: McCormack, Brian James, By Kircaldy, Fife KY2 6QS (GB)
(72) Inventor: McCormack, Brian James, By Kircaldy, Fife KY2 6QS (GB)
(74) Representative: Spencer, Michael David
(86) International application number: PCT/GB2018/050957
(87) International publication number: WO 2018/189531

(56) References cited:
- US-A- 2 703 083
- US-B1- 6 269 820

## Description

The present invention relates to a wound dressing, surgical tape, cannula dressing, bandage or plaster.

Wound dressings, surgical tape, bandages and plasters are widely used to cover wounds, hold together wounds or cover simple cuts. Previously proposed wound dressings, surgical tape, bandages and plasters have used sticky fabric, adhesive plastics film and suchlike to attach them to a patient's skin. In the case of a wound dressing or plaster the sticky fabric, adhesive plastic film holds an absorbent pad, often impregnated with anti-septic or other topical medicine, on the wound or skin of the patient. Such pads are used to absorb leaking fluid or protect against foreign bodies entering the wound and necessarily the adhesion is designed to hold the pad securely against the skin. It is normally necessary to replace such plasters or dressings regularly due to leakage of fluid from the wound with a fresh dressing or a different type of dressing. The replacement of such a wound dressing or plaster involves tugging off the old plaster or dressing thereby often causing further damage to the wound. In the case of surgical tape for instance is often used in operations to hold people's eyes shut during an operation and removing the tape particularly from elderly patients causes distress.

A cannula is a hollow tube with a sharp, retractable inner core that can be inserted into a vein, an artery, or another body cavity. It is generally covered with a specific dressing to hold it in place and stop the patient accidentally disturbing it.

When removing a traditional cannula dressing, there can be a pain and discomfort due to the skin being pulled whilst the cannula is still in place in the vein.

It is an aim of the present invention to provide a wound dressing, surgical tape, cannula dressing, bandage or plaster which seeks to overcome these previously mentioned disadvantages.

Accordingly the present invention is directed to a wound dressing, surgical tape or plaster in which the adhesive part comprises adhesive soluble paper. The water-soluble part can be easily removed via wiping with a wet sponge/cloth or even running water leaving little or no residue.

In a preferred embodiment the wound dressing or plaster, the adhesive soluble paper part covers an adsorbent pad. This provides the advantage that the adhesive paper attaches to the absorbent pad as well as to the skin of the person being treated. The soluble paper can then be removed without disturbing the absorbent pad or dressing. This can then be gently removed with appropriate care to limit damage to the wound.

Advantageously the wound dressing, surgical tape or plaster has a removable film/foil covering its adhesive side before use. This enables the film/foil to be removed before use of the wound dressing, surgical tape or plaster.

Preferably the dissolvable paper can be printed or written or on one side conveying information such as the procedure in process or instructions for the removal of the paper.

Advantageously the wound dressing is covered by a removable water resistance film. This provides the advantage that if the wound dressing is accidentally splashed with water while in use it does not start to dissolve.

In a preferred embodiment the film is secured to that intended topside of the wound dressing via adhesive that is relatively weak to enable easy removal. Advantageously the film as a corner or edge that bears no adhesive in order to enable easy peel off before removal of the dressing.

Preferably the absorbent pad of the wound dressing is made of water soluble material. The water-soluble material may be similar to or the same as that of the rest of the wound dressing or of a different material. This provides the additional advantage that when removing the top of the wound dressing the absorbent pad can also be gently washed away thus dealing with any adhesion of the pad to sutures or the like.

Preferably, the wound dressing is a cannula dressing. This dressing is advantageous for a short term treatment and when the patient is bed-ridden, especially for burns injuries.

Preferably, the cannula dressing is made from two forms of soluble material.

Advantageously, the cannula dressing can be made to any size or shape.

In a preferred embodiment, the cannula dressing can be secured to the cannula site.

Preferably, the cannula dressing can be flushed or washed away by water/saline spray or wet sponge.

A bandage of elongate form in which sections of the bandage can be of a woven or non-woven fabric, comprising multiple sections, wherein the sections are held together via water-soluble fibre material. This provides the advantage that if a wound dressing is covered by a standard elongate bandage the bandage can be removed carefully particularly when soiled in sections.

The sections can be sewn together with water soluble thread. The sections can be joined together by non-woven soluble material such as paper fibres which are joined with either each section of the bandage by needling or alternatively adhesive. This provides the advantage of a long bandage being produced with multiple sections which can easily be broken apart by water. In the case of the sections of soluble material this gives a significant break between the sections of bandage.

Examples of wound dressings made in accordance with the present invention will now be described hereinbelow with reference to the accompanying drawings, in which:

- Figure 1: shows a top plan view of a plaster;
- Figure 2: shows a cross-sectional side view of the plaster in Figure 1;
- Figure 3: shows a bottom view of a plaster with a removable film present;
- Figure 4: shows a top plan view of a cannula dressing; and
- Figure 5: shows top views of bandages.

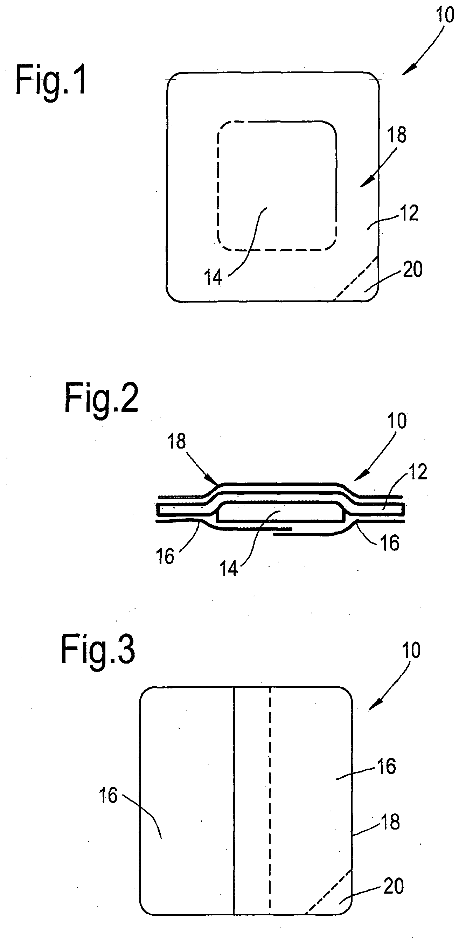

Figure 1 shows a plaster 10. In this instance the plaster is a roughly square shape however obviously different shapes are equally possible. The plaster 10 comprises on its top side a dissolvable paper 12. Dissolvable paper made from Sodium Carboxy Methyl Cellulose and wood/wooden pulp, such as that produce by Aquasol Paper is suitable. In the centre of the plaster 10 there is seen a raised portion under which is present an absorbent pad 14. The absorbent pad 14 can be a conventional pad or alternatively can be made from absorbent soluble material. The plaster 10 can be covered by a water resistant film 18 on its intended top surface. The film 18 has a corner 20 with no adhesive.

Figure 2 shows a cross-section of the plaster 10. The top side the plaster 10 comprises the dissolvable paper 12, which is covered by the water resistant film 18 lightly adhered to the dissolvable paper 12. The underside of the dissolvable paper 12 has adhesive material on it. The adhesive is selected from a number of readily dissolvable adhesives. The adhesive holds the absorbent pad 14 to the dissolvable paper 12. Both the dissolvable paper 12 and the absorbent pad 14 are covered by a foil 16 which covers the bottom of the plaster 10 which faces the patient's skin.

Figure 3 shows the bottom of the plaster 10 in which it can be seen that the foil 16 has two parts which cover the bottom to the plaster 10 with one half over lapping the other to enable removal of the film 16.

For supply to the public the plaster 10 may be placed in a packet which is sterile. The paper can be selected from ones that are hyper allergenic in order to ensure least irritation to patient's skin.

When the plaster 10 is in use the film 16 is removed from the plaster 10 and the plaster 10 is applied to the cut or wound. When it is desired to remove the plaster 10, the film 18 if presence is peeled back. The plaster 10 is either treated with running water or alternatively has a wet cloth or sponge applied to remove the dissolvable paper and with care. This will not remove the absorbent pad 14. However if the absorbent pad is also made of dissolvable material this can be removed at the same time. The absorbent pad 14 can then be removed with as little disturbance of the wound as possible.

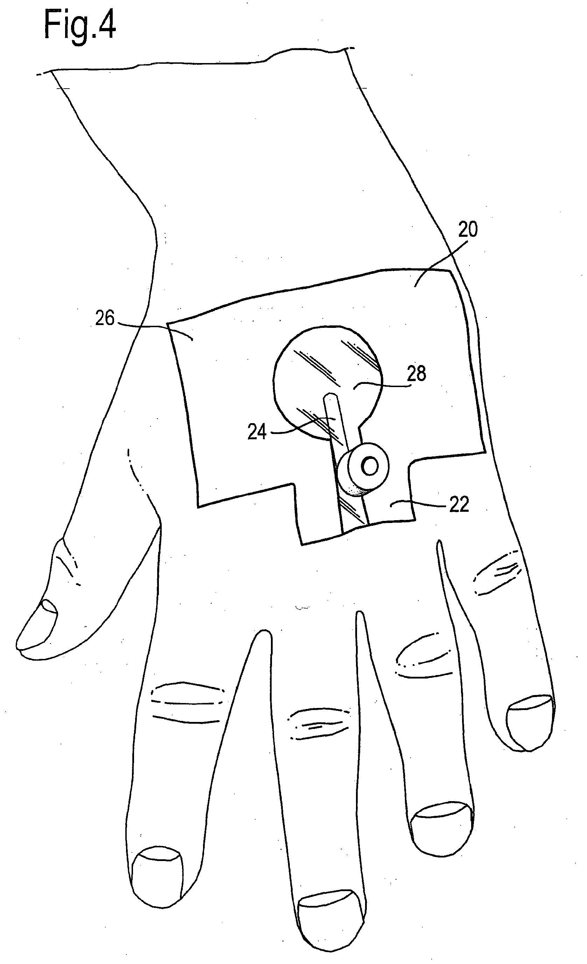

Figure 4 shows a cannula dressing 20. In this instance, the plaster is a roughly parallelogram shape with an elongated bottom part 22 to cover the cannula 24 but obviously different shapes are equally possible. The cannula dressing 20 comprises on its top side a dissolvable paper 26. Dissolvable paper made from Sodium Carboxy Methyl Cellulose and wooden pulp, such as that produce by Aquasol Paper. In the centre of the cannula dressing 20 there is a transparent part 28 which enables the cannula 24 to be seen. In this instance, the transparent part 28 is circular but obviously different shapes are equally possible. The underside of the dissolvable paper 26 has adhesive material on it. The adhesive is selected from a number of readily dissolvable adhesives. The cannula dressing holds the cannula in place on, in this instance, the left hand. When it is necessary to remove the cannula, a wet sponge, shower or a spray can be used to dissolve the paper, thus allowing the easy removal of the cannula.

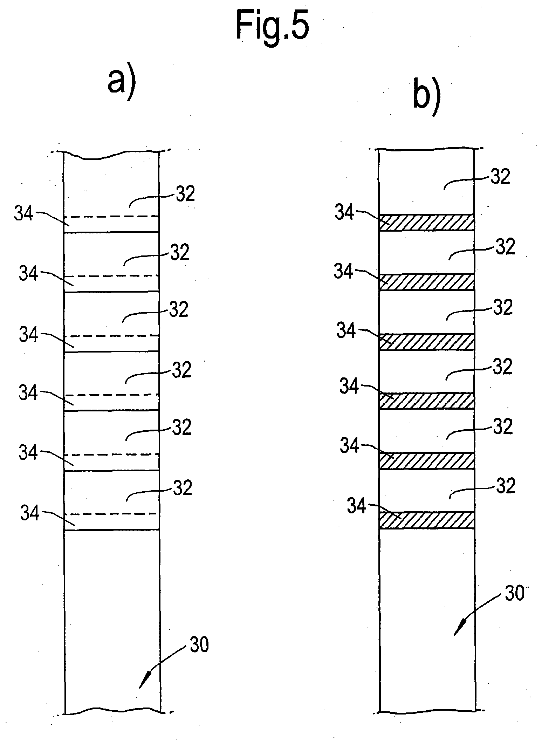

Figure 5 shows to plan views of an elongate bandage 30. Each bandage 30 is comprised of a number of sections 32. The bandage 30 can be of a woven cloth fabric or alternatively of a non-woven fabric. The size of each section 32 of the bandage 30 can be varied and will be dependent upon the intended application. In Figure 5a the sections 32 are sewn together with soluble thread at points 34. In Figure 5b there exists a section of soluble material 34 which is joined to each section of bandage 32. The joint between sections here can be achieved nonwoven fashion by needling or alternatively by adhesive or other fixing means. The soluble material may be soluble paper or non-woven soluble paper/wood fibre.

Water is applied to the bandage 30 breaking the sections 32 and thus allowing careful removal of each section 32 to occur. Obviously the bandage of Figure 5b allows discrete breaks in the bandage 30 and may be suitable for certain purposes where the breakup of the elongate bandage 30 is more required.

## Claims

1. A wound dressing, surgical tape or plaster comprising an absorbent pad and an adhesive part in which the adhesive part is an adhesive dissolvable paper.

2. A wound dressing, surgical tape or plaster according to claim 1, in which the adhesive dissolvable paper part covers an adsorbent pad.

3. A wound dressing, surgical tape or plaster according to any preceding claim, further comprising a removable film or foil covering its adhesive side before use.

4. A wound dressing, surgical tape or plaster according to any preceding claim, in which the adhesive dissolvable paper is printed or written or on one side conveying information.

5. A wound dressing surgical tape or plaster according to any preceding claim, further comprising a removable water resistance film.

6. A wound dressing surgical tape or plaster according to claim 5, in which the removable water resistance film is secured to the topside of the wound dressing surgical tape or plaster via adhesive.

7. A wound dressing surgical tape or plaster according to any preceding claim, in which the absorbent pad of the wound dressing is made of water soluble material.

8. A wound dressing surgical tape or plaster according to any preceding claim, in which the wound dressing surgical tape or plaster is a cannula dressing.

9. A wound dressing surgical tape or plaster according to claim 8, in which the cannula dressing is made from two forms of soluble material.

10. A wound dressing surgical tape or plaster according to claim 8 or claim 9, in which the cannula dressing can be made to any size or shape.

11. A wound dressing surgical tape or plaster according to any preceding claims 8 to 10, in which the cannula dressing can be secured to the cannula site.

## Patentansprüche

1. Wundversorgung, chirurgisches Klebeband oder Pflaster, das ein absorbierendes Polster und ein haftendes Element umfasst, wobei das haftende Element ein haftendes auflösbares Papier ist.

2. Wundversorgung, chirurgisches Klebeband oder Pflaster nach Anspruch 1, wobei das haftende auflösbare Papier ein absorbierendes Polster bedeckt.

3. Wundversorgung, chirurgisches Klebeband oder Pflaster nach einem der vorhergehenden Ansprüche, das ferner eine abnehmbare Schicht oder Folie umfasst, die die haftende Seite vor der Verwendung bedeckt.

4. Wundversorgung, chirurgisches Klebeband oder Pflaster nach einem der vorhergehenden Ansprüche, wobei das haftende auflösbare Papier bedruckt oder beschrieben ist oder auf einer Seite Informationen trägt.

5. Wundversorgung, chirurgisches Klebeband oder Pflaster nach einem der vorhergehenden Ansprüche, das ferner eine abnehmbare wasserbeständige Folie umfasst.

6. Wundversorgung, chirurgisches Klebeband oder Pflaster nach Anspruch 5, wobei die abnehmbare wasserbeständige Folie an der Oberseite der Wundversorgung, des chirurgischen Klebebands oder des Pflasters durch einen Klebstoff befestigt ist.

7. Wundversorgung, chirurgisches Klebeband oder Pflaster nach einem der vorhergehenden Ansprüche, wobei das absorbierende Polster der Wundversorgung aus wasserlöslichem Material hergestellt ist.

8. Wundversorgung, chirurgisches Klebeband oder Pflaster nach einem der vorhergehenden Ansprüche, wobei die Wundversorgung, das chirurgische Klebeband oder das Pflaster eine Kanülenversorgung ist.

9. Wundversorgung, chirurgisches Klebeband oder Pflaster nach Anspruch 8, wobei die Kanülenversorgung aus zwei Formen eines löslichen Materials hergestellt ist.

10. Wundversorgung, chirurgisches Klebeband oder Pflaster nach Anspruch 8 oder Anspruch 9, wobei die Kanülenversorgung in beliebiger Größe oder Form hergestellt werden kann.

11. Wundversorgung, chirurgisches Klebeband oder Pflaster nach einem der vorhergehenden Ansprüche 8 bis 10, wobei die Kanülenversorgung an der Kanülenposition befestigt werden kann.

## Revendications

1. Pansement pour plaies, bande chirurgicale ou plâtre comprenant un tampon absorbant et une partie adhésive dans laquelle la partie adhésive est un papier adhésif soluble.

2. Pansement, bande chirurgicale ou plâtre selon la revendication 1, dans lequel /laquelle la partie adhésive en papier soluble recouvre un tampon adsorbant.

3. Pansement pour plaies, bande chirurgicale ou plâtre selon l'une quelconque des revendications précédentes, comprenant en outre un film ou une feuille amovible recouvrant sa face adhésive avant utilisation.

4. Pansement pour plaies, bande chirurgicale ou plâtre selon l'une quelconque des revendications précédentes, dans lequel /laquelle le papier adhésif soluble est imprimé ou écrit ou communiquant des informations sur une face.

5. Pansement pour plaies bande chirurgicale ou plâtre selon l'une quelconque des revendications précédentes, comprenant en outre un film amovible résistant à l'eau.

6. Pansement pour plaies bande chirurgicale ou plâtre selon la revendication 5, dans lequel /laquelle le film amovible résistant à l'eau est fixé à la face supérieure du pansement chirurgical pour pansement via un adhésif.

7. Pansement pour plaies bande chirurgicale ou plâtre selon l'une quelconque des revendications précédentes, dans lequel /laquelle le tampon absorbant du pansement pour plaies, bande chirurgicale ou plâtre est constitué d'un matériau soluble dans l'eau.

8. Pansement pour plaies bande chirurgicale ou plâtre selon l'une quelconque des revendications précédentes, dans lequel /laquelle le pansement pour plaies, bande chirurgicale ou plâtre est un pansement pour canule.

9. Pansement pour plaies bande chirurgicale ou plâtre selon la revendication 8, dans lequel /laquelle le pansement pour canule est fabriqué à partir de deux formes de matériau soluble.

10. Pansement pour plaies bande chirurgicale ou plâtre selon la revendication 8 ou la revendication 9, dans lequel /laquelle le pansement pour canule peut être réalisé dans n'importe quelle taille ou forme.

11. Pansement pour plaies bande chirurgicale ou plâtre selon l'une quelconque des revendications précédentes 8 à 10, dans lequel /laquelle le pansement pour canule peut être fixé au site de la canule.
